# EUROPEAN PATENT APPLICATION

(11) **EP 4 446 305 A1**
(43) Date of publication of application: **16.10.2024**
(21) Application number: 23168022.4
(22) Date of filing: 14.04.2023
(51) Int. Cl.: C07C 37/16, C07C 39/14

(54) **AN ALKYLATION PROCESS OF NAPHTHOL**

(71) Applicant: DSM IP Assets B.V., 6221 BE Maastricht (NL)
(72) Inventor: MONACO, Claudio, 40126 Bologna (IT); CAVANI, Fabrizio, 40126 Bologna (IT); TABANELLI, Tommaso, 40126 Bologna (IT); BONRATH, Werner, 4303 Kaiseraugst (CH); SCHUETZ, Jan, 4303 Kaiseraugst (CH)
(74) Representative: dsm-firmenich IP

(57) **Abstract**

The present invention provides a process for producing a compound of formula (I). According to the process of the present invention, the compound of formula (I) can be produced in a high conversion and yield an in a stable way, 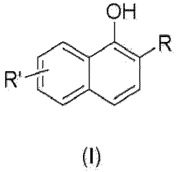 wherein R is alkyl, preferably C₁₋₆ alkyl, more preferably methyl, R' is H or 1-6 alkyls, more preferably C₁₋₆ alkyl, connected to any position on the naphthalene ring.

## Description

### Technical Field

The present invention is related to an improved process for alkylation of naphthol. Particularly, the present invention is related to a process for alkylation of 1-naphthol to 2-methyl-1-naphthol.

### Background of the Invention

Menadione (2-methyl-1,4-naphthoquinone or vitamin K₃) is commonly used as a blood clotting agent and is also the key intermediate in the production of the other group of K vitamins. The traditional way of producing menadione in an industrial scale consists of the stoichiometric oxidation of 2-methylnaphthalene by CrO₃ in sulfuric acid. Yield and selectivity are typically around 50%. This method produces also around 18 kg of toxic inorganic waste per 1 kg of target product. The environmental and safety problems linked to the disposal of the toxic waste has stimulated the search for alternative synthesis methods that do not use chromium as stoichiometric oxidizer.

Among the proposed alternatives, 2-methyl-1-naphthol represents a different starting point from 2-methylnaphthalene, and it can be oxidized more simply without the use of any toxic substances.

Klemm *et al.* published the results of the reaction of naphthol and their derivatives with methanol over alumina at different reaction temperatures. In the case of 1-naphthol as substrate, the corresponding mono-methylated compound, 2-methyl-1-naphthol, was obtained with a maximum yield of 37%. (see: Klemm, L.H. et al., J. Org. Chem., 1970, 33(4), 1480-1488)

Kulkami *et al.* also evaluated the efficiency of lanthanum modified Y zeolite (MLaY, M = Na, Ca, Mg, Fe, Ni or Co, with 5 wt.% La and 5 wt.% M) in naphthol methylation with methanol. The study results showed that LaY promoted with alkali and alkaline metals favored C-methylation, mainly the 2-methyl-1-naphthol in yields of 52-58%. (see: Kulkarni, S.J, et al., Microporous Mesoporous Mater., 1998, 21(1-3), 53-57)

Hematite (Fe₂O₃) was also investigated for the selective methylation of 1-naphthol, giving 1-naphthol conversion of 57.5% and 100% selectivity of 2-methyl-1-naphthol. (see: Inoue, et al., Chem. Pharm. Bull. (Tokyo), 1976, 24(9), 2199-2203)

More recently, *Cavani et al.* reported excellent results by using MgO, Fe₂O₃ and mixed oxides based on magnesium and iron for the gas-phase alkylation of naphthol with methanol. Thanks to a mixed oxide (Mg/Fe/O) characterized by a Mg/Fe atomic ratio equal to 0.75 they obtained a conversion of 1-naphthol equal to 98% with a yield in 2-methyl-1-naphthol of 96% (see: WO 2004014832 A2). However, the patent does not show the trends of conversion and yields of the reaction as the time on stream.

Accordingly, there is still demand in further investigating the process for methylation of naphthol compounds in term of conversion and yield and stability as the time on stream.

### Description of the Figures

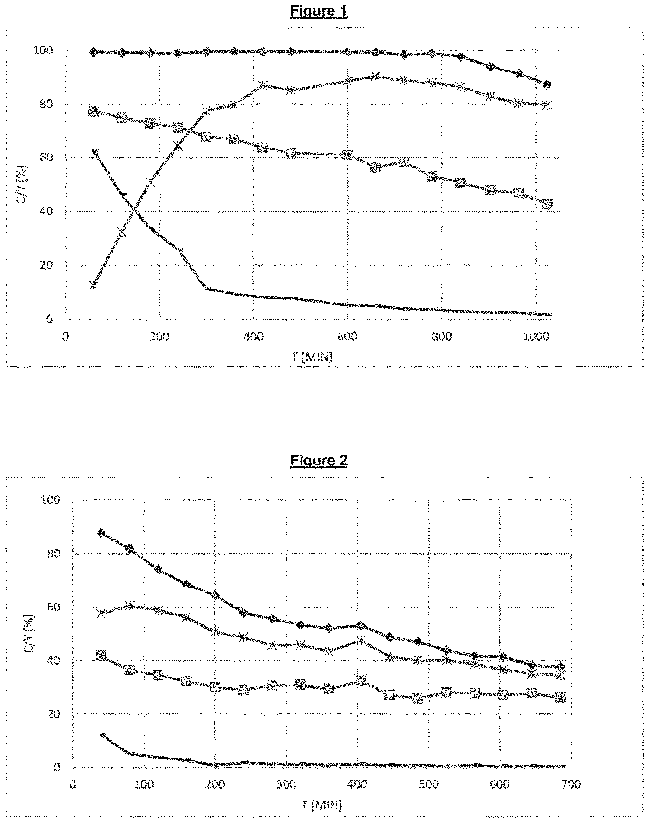

**Figure 1** indicates conversion and yield of catalytic methylations of 1-naphthol with the prior art Mg/Fe/O catalyst (Mg/Fe = 0.75). In the figure, "C/Y" is conversion and yield, "T" is time on stream, the line " 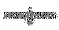 " represents the change of conversion of naphthol as the time on stream, the line " 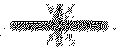 " represents the change of yield of 2-methylnaphthol as the time on stream, the line " 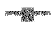 " represents the change of conversion of methanol as the time on stream, and the line " 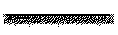 " represents the change of yield of 2,4-dimethyl naphthol as the time on stream.
**Figure 2** indicates conversion and yield of catalytic methylations of 1-naphthol with the prior art Mg/Fe/O catalyst (Mg/Fe = 0.75) at higher concentration of naphthol (6.64 mol%). In the figure, "C/Y" is conversion and yield, "T" is time on stream, the line " 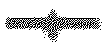 " represents the change of conversion of naphthol as the time on stream, the line " " represents the change of yield of 2-methylnaphthol as the time on stream, the line " 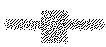 " represents the change of conversion of methanol as the time on stream, and the line " " represents the change of yield of 2,4-dimethyl naphthol as the time on stream.

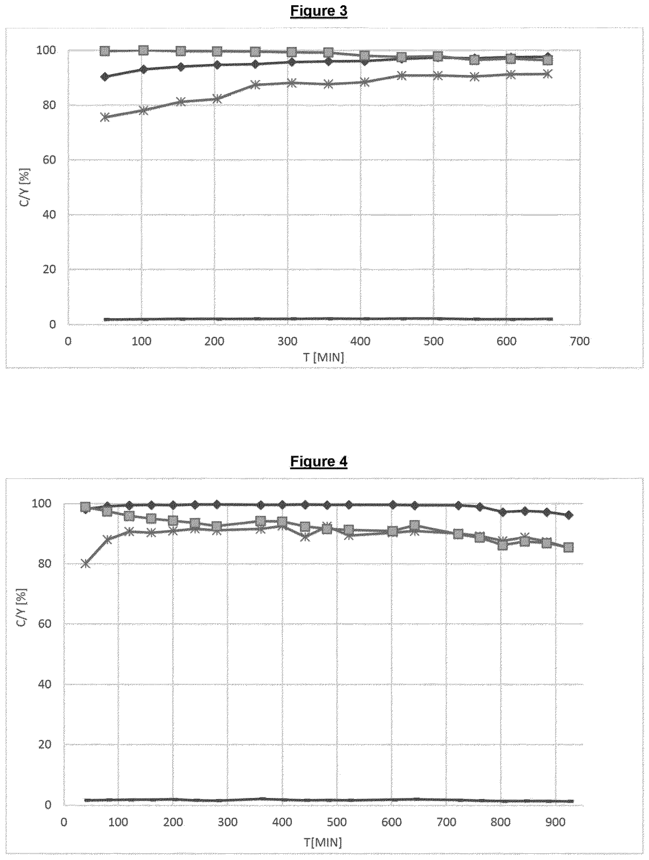

**Figure 3** indicates conversion and yield of catalytic methylations of 1-naphthol with the ZnFe₂O₄ catalyst according to the present invention. In the figure, "C/Y" is conversion and yield, "T" is time on stream, the line " 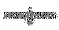 " represents the change of conversion of naphthol as the time on stream, the line " " represents the change of yield of 2-methylnaphthol as the time on stream, the line " 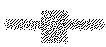 " represents the change of conversion of methanol as the time on stream, and the line " " represents the change of yield of 2,4-dimethyl naphthol as the time on stream.
**Figure 4** indicates conversion and yield of catalytic methylations of 1-naphthol with the ZnFe₂O₄ catalyst according to the present invention at higher concentration of naphthol (6.3 mol%), In the figure, "C/Y" is conversion and yield, "T" is time on stream, the line " 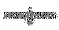 " represents the change of conversion of naphthol as the time on stream, the line " 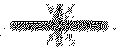 " represents the change of yield of 2-methylnaphthol as the time on stream, the line " 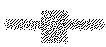 " represents the change of conversion of methanol as the time on stream, and the line " " represents the change of yield of 2,4-dimethyl naphthol as the time on stream.

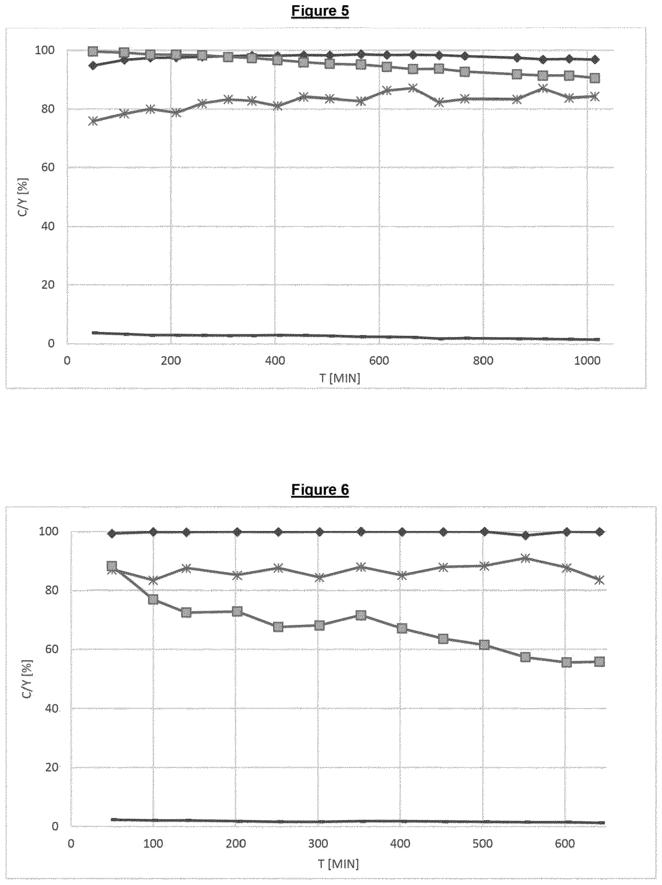

**Figure 5** indicates conversion and yield of catalytic methylations of 1-naphthol with the Zn/Fe/O catalyst (Zn/Fe = 3) according to the present invention. In the figure, "C/Y" is conversion and yield, "T" is time on stream, the line " 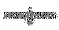 " represents the change of conversion of naphthol as the time on stream, the line " 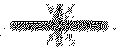 " represents the change of yield of 2-methylnaphthol as the time on stream, the line " 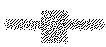 " " represents the change of conversion of methanol as the time on stream, and the line " " represents the change of yield of 2,4-dimethyl naphthol as the time on stream.
**Figure 6** indicates conversion and yield of catalytic methylations of 1-naphthol with the Zn/Fe/O catalyst (Zn/Fe = 3) according to the present invention at a decreased temperature (360°C). In the figure, "C/Y" is conversion and yield, "T" is time on stream, the line " 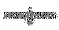 " represents the change of conversion of naphthol as the time on stream, the line " " represents the change of yield of 2-methylnaphthol as the time on stream, the line " 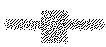 " represents the change of conversion of methanol as the time on stream, and the line " " represents the change of yield of 2,4-dimethyl naphthol as the time on stream.

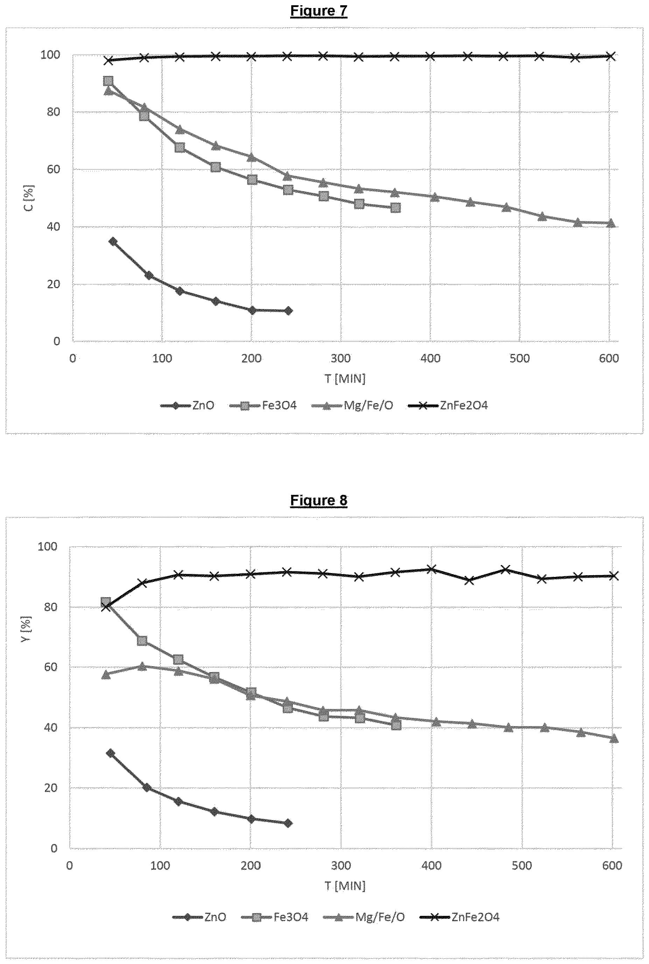

**Figure 7** indicates conversion of catalytic methylations of 1-naphthol with four catalysts: ZnO, Fe₃O₄, Mg/Fe/O catalyst and ZnFe₂O₄ catalyst. In the figure, "C" is conversion of 1-naphthol, and "T" is time on stream.
**Figure 8** indicates yield of catalytic methylations of 1-naphthol with four catalysts: ZnO, Fe₃O₄, Mg/Fe/O catalyst and ZnFe₂O₄ catalyst. In the figure, "Y" is yield of 1-naphthol, and "T" is time on stream.

### Summary of the Invention

The present invention provides a process for alkylation of a naphthol compound of formula (II) to a compound of formula (I) in the presence of a catalyst containing a mixed oxide of zinc and iron, 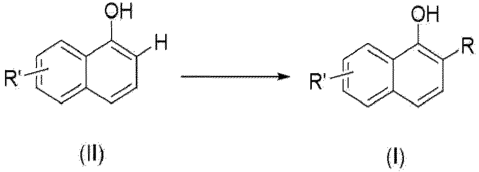 wherein R is alkyl, preferably C₁₋₆ alkyl, more preferably methyl; R' is H or 1-6 alkyls, more preferably C₁₋₆ alkyl, connected to any position on the naphthalene ring.

According to the process of the present invention, the compound of formula (I) can be produced in a high conversion and yield, and the used catalyst is surprisingly stable as the time on stream in the reaction.

### Detailed description of the Invention

In the present invention, the term "alkyl" refers to a saturated hydrocarbon group that may be straight-chain or branched, preferably having 1 to 6 carbons, i.e. C₁₋₆ alkyl. In some embodiments, the alkyl group contains from 1 to 5 carbon atoms or from 1 to 4 carbon atoms, or from 1 to 3 carbon atoms. Examples of the alkyl moieties include, but are not limited to, chemical groups such as methyl, ethyl, n-propyl, isopropyl, n-butyl, s-butyl, and t-butyl.

Particularly, the present invention provides a process for producing a compound of formula (I) comprising alkylation of the compound of formula (II) in the presence of a catalyst to obtain the compound of formula (I), 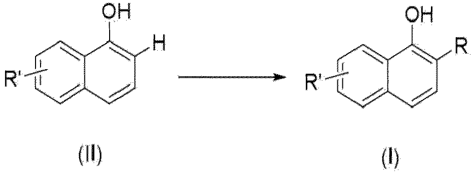
wherein R and R' is independently defined as above, and
wherein the catalyst contains a mixed oxide of zinc and iron.

In the present invention, the alkylation is achieved by contacting a mixture that comprises the compound of formula (II) and an alcohol in gas phase with the catalyst.

The alcohol may be an aliphatic, primary or secondary alcohol, either linear or branched, and with 1 to 6 carbon atoms in the hydrocarbon chain, or mixture thereof. Preferred examples of the alcohol are methanol, ethanol, 2-propanol, 1-propanol, 1-butanol and 2-butanol. The more preferred examples are methanol, ethanol and 2-propanol, even more preferably methanol.

The alcohol may be used in an amount of from 1 mol to 50 moles, preferably from 3 moles to 20 moles, more preferably from 5 moles to 10 moles, per 1 mol of the compound of formula (II).

In the present invention, the oxide of zinc is zinc oxide, and the oxide of iron is iron oxides. Examples of the iron oxides are Fe₂O₃, Fe₃O₄ and FeO. The zinc oxide and iron oxides may be used in any physical form thereof and may be provided as separate entities or mixtures in the process of the present invention.

Advantageously, the catalyst according to the present invention has an atomic ratio between zinc and iron (Zn/Fe) in a range from 0.1 to10, preferably from 0.25 to 5, more preferably from 0.5 to 4 such as 0.5, 0.75, 1, 2, 3 and 4.

Preferably, the catalyst according to the present invention is represented by the formula of ZnₓFe_{y}O_{z} wherein x is an integer from 1 to 3, preferably is 1; y is an integer from 1 to 4, preferably is 2; and z is an integer from 1 to 10, preferably is 4.

The catalyst containing a mixed oxide of zinc and iron may be prepared from raw materials containing iron and raw materials containing zinc.

The raw materials containing iron may be salts or oxides of iron (ferric and ferrous). Preferably, the raw materials containing iron can be selected from the group consisting of ferric oxide, ferrous oxide, maghemite, magnetite, hematite, and iron salts such as iron chlorides and iron nitrates, and mixtures thereof. More preferably, the raw materials containing iron are ferric oxide and/or ferrous oxide.

The raw materials containing zinc may be salts or oxides of zinc. Examples of the raw materials containing zinc include but are not limited to zinc oxide, zinc chloride and zinc nitrate.

The catalyst used in the process of the present invention may be prepared by any method such as precipitation and mechanical mixing known in the art, preferably by the method as disclosed in WO 2004/014832 A.

Advantageously, after its preparation but prior to its use, the catalyst according to the present invention may be subjected to a thermal treatment performed in an atmosphere of air, at a temperature from 450 °C to 700 °C, such as 450 °C, 500 °C, 600 °C and 700 °C.

Surprisingly it has been found that, in the process of the present invention, the catalyst as described herein provides not only better conversion and yield, but also unexpected higher stability as the time on stream compared to the individual metals and the catalysts known in the art.

In the process of the present invention, the total contact time, understood as the ratio between the volume of catalyst and the total volumetric flow-rate of the gas phase (containing the compound of formula (II) and the alcohol and gas such as molecular nitrogen), may be from 0.1 seconds to 10 seconds, preferably from 0.5 to 5 seconds such as 0.5, 1, 2, 3, 4 and 5 seconds.

The molar ratio between the mixture (the compound of formula (II) such as naphthol and the alcohol) in the gas phase and the total feed to the reactor (the mixture and carrier gas) may be from 5% to 80%, preferably from 10% to 60%, more preferably from 15% to 50% such as 15%, 20%, 25%, 30%, 35%, 40%, 45% and 50%.

In the process of the present invention, the reaction may be carried out at from 300 °C to 500 °C, preferably from 350°C to 450 °C, more preferably from 360 °C to 390 °C.

In the process of the present invention, the reaction may be carried out at the pressure of from 0.1 bar to 50 bar, preferably from 1 bar to 10 bar.

The obtained compound of formula (I) may be used directly to the next step for producing, for example, menadione or isolated and purified by known measure.

Surprisingly, it is discovered that the compound of formula (II) such as 1-naphthol can be alkylated in a high yield and selectivity to produce the compound of formula (I) such as 2-methyl-1-naphthol. More surprisingly, the catalyst used in the reaction is not deactivated and thus the reaction is unexpectedly stable as the time on stream in the reaction.

The process of the present invention will be further illustrated and explained by the following examples.

### Examples

**The preparation of the catalyst was performed according to the following method:** a weighted amount of Zn(NO₃)₂ • 6H₂O and Fe(NOs)s • 9H₂O is dissolved in demineralized water to provide a homogeneous solution of cations Zn²⁺ and Fe³⁺ in a chosen atomic ratio. The dissolved quantity might correspond, for example, to the provision of a 1 M concentration of cations. Separately, Na₂CO₃ is dissolved in demineralized water (1.2 M). The first solution is dropped into the second one, keeping the temperature at 50 °C under stirring. The pH of the solution is kept constant at approximately 10 by adding NaOH 3 M. The result is a precipitate, and slurry digestion is allowed for approximately 1 hour. The precipitate is then filtered and washed with abundant demineralized H₂O. The precipitate is dried at 90 °C for one night and then subjected to heat treatment by gradual heating in static air. The temperature is raised from 120 °C to a final temperature than can be between 450 °C and 700 °C, with a thermal gradient of 5 °C/min. The final temperature is maintained for 5 hours to obtain the catalyst powder.

**Catalytic methylations** were performed in using a lab-scale tubular glass reactor operating at atmospheric pressure, by loading 1 mL of catalyst. The liquid mix of methanol and 1-naphthol is prepared to have a methanol/naphthol molar ratio. These tests were performed at 390 °C, although lower temperatures such as 360 °C were used in some cases.

The liquid mix is injected with the pump at a rate between 0.35 and 1.40 mL/h, while the nitrogen flow is between 20 and 25 mL/min (measured at 20 °C and at atmospheric pressure). These two values are set to have a contact time (t) over the catalyst at the reaction temperature (calculated by considering the volumetric flow of both the organic and the inert dilutant) between 0.5 and 2 s and the molar ratio between the mixture of reagents (in gas phase) and the total feed to reactor between 13% and 40%.

The collection of the reaction products is extended for a definite time (typically 1 hour), finally everything is collected in 50 ml of acetonitrile. The analysis is then performed off-line by a gas chromatograph equipped with an Agilent HP-5 column. The reported data (yields, conversion, selectivity) are calculated on a molar basis.

### Example 1. Naphthol methylation over the prior art Mg/Fe/O catalyst (Mg/Fe = 0.75)

A catalyst containing iron and magnesium with Mg/Fe ratio equal to 0.75 was prepared according to the procedures as disclosed in Example 9 of PCT patent publication WO2004014832A2.

A methylation was performed with the catalyst as described above under the reaction conditions as below:
- Reaction temperature (T) = 390°C
- Methanol/naphthol molar ratio (MeOH/Naph) = 10:1
- Contact time (t) =1 s
- mol percentage of organic mixture (%mol organic) = 13 %
- mol percentage of naphthol (%mol naphthol) = 1.18 %
- N₂ flow = 23,2 ml/min; and
- Pump rate = 0.4 ml/h.

The results are shown in Figure 1. As indicated, in the first five hours of the reaction, the yield of desired product 2-methylnaphthol was low. In addition, the yield of 2-methyl-naphthol starts to drop together with the conversion of naphthol after about 12 h, and methanol was decomposed as the time on stream.

Another test was performed with the same Mg/Fe/O catalyst, by increasing the %vol of naphthol fed to the reactor. The reaction conditions are as below.
- T = 390°C
- MeOH/Naph = 5:1
- t = 1s
- %mol organic = 36.0 %
- %mol naphthol = 6.0 %
- N₂ flow = 16.5 ml/min; and
- Pump rate = 1.4 ml/h.

The results are shown in Figure 2. As indicated in Figure 2, at more "stressful" conditions of high concentration of naphthol, the Mg/Fe/O catalyst failed to replicate the performance seen in the previous test (Figure 1). The yield of 2-methyl-naphthol was never stable and always below 60%.

### Example 2. Naphthol methylation over the ZnFe₂O₄ catalyst according to the present invention

A catalyst was prepared according to the method described above in order to obtain a Zn/Fe ratio, expressed as the atomic ratio between the two elements, equal to 0.5. The dried material was finally subjected to a thermal treatment in air at 500°C for 5 hours. The specific surface area was 25 m²/g.

The catalytic methylation was performed as described above with the following reaction conditions:
- T = 390°C
- MeOH/Naph = 5:1
- t =1 s
- %mol organic = 17.9 %
- %mol naphthol = 2.9 %
- N₂ flow = 21 ml/min; and
- Pump rate = 0.7 ml/h.

The results are shown in Figure 3. As indicated, the reaction was stable and the conversion of naphthol was also total throughout the time on stream, and the yield of 2-methyl-1-naphthol quickly reached values above 80% in about 100 mins and above 90% in 200 mins.

Another reaction was performed under the same condition but higher concentration of organic in the feed. The reaction conditions were as below:
- T = 390°C;
- MeOH/Naph = 5:1;
- t =1 s
- %mol organic = 37.5 %;
- %mol naphthol = 6.3 %;
- N₂ flow = 16.8 ml/min; and
- Pump rate = 1.4 ml/h.

The results are shown in Figure 4. As indicated, stabilization of naphthol conversion and yield of 2-methyl naphthol were achieved in little more than an hour and their value were 99% and 90% respectively, even at more "stressful" conditions. Moreover, these values were stable for almost 11 hours of the time on stream despite 40 vol% organic was being fed. It's worth noting that the selectivity was very high throughout the time on stream. This test shows that the ZnFe₂O₄ is able to perform even in these conditions without a significant drop in performance.

### Example 3. Naphthol methylation over Zn/Fe/O catalyst (Zn/Fe=3) according to the present invention

A catalyst was prepared according to the method described above in order to obtain a Zn/Fe ratio, expressed as the atomic ratio between the two elements, equal to 3. The dried material was finally subjected to a thermal treatment in air at 500 °C for 5 hours.

The catalytic methylation was performed as described above with the following reaction conditions:
- T = 390°C
- MeOH/Naph = 5:1
- t =1 s
- %mol organic = 17 %
- %mol naphthol = 2.8 %
- N₂ flow = 21 ml/min; and
- Pump rate = 0.65 ml/h.

The results are shown in Figure 5.

Another catalytic methylation was performed as described above at decreased reaction temperature. The Reaction conditions are as below:
- T = 360°C
- MeOH/Naph = 5:1
- t =1 s
- %mol organic = 17.7 %
- %mol naphthol = 2.9 %
- N₂ flow = 22 ml/min; and
- Pump rate = 0.7 ml/h.

The results are shown in Figure 6.

As indicated, the conversion of naphthol was practically total throughout the time on stream and 2-methyl-1-naphthol was always the main product with a yield greater than 80%. The selectivity was also good because there were no consecutive methylation products.

### Example 4. Comparison of naphthol methylation with different catalysts

The catalytic methylation was performed as described above with four catalysts: ZnO, Fe₃O₄, Mg/Fe/O catalyst and ZnFe₂O₄ catalyst. the Mg/Fe/O catalyst containing iron and magnesium with Mg/Fe ratio equal to 1 was prepared according to the procedures as described in Example 1. The ZnFe₂O₄ catalyst was prepared according to the procedures as described in Example 2.

The reaction condition was as below:
- T = 390°C
- MeOH/Naph = 5:1
- t =1 s
- %mol organic = 36 %
- %mol naphthol = 6 %
- N₂ flow = 16.5 ml/min; and
- Pump rate = 1.4 ml/h.

The results are shown in Figures 7 and 8.

As indicated in Figure 7, the naphthol conversion quickly reached the value of almost 100% and maintained stable in the next 10 hours when the ZnFe₂O₄ catalyst was used. In contract, the naphthol conversion with the catalyst Mg/Fe/O and Fe₃O₄ reached the highest point in 1 hour but immediately dropped down as the time on stream.

In addition, as indicated in Figure 8, the ZnFe₂O₄ catalyst provided yield of the desired compound 2-methylnaphthol which is much higher and stabler compared to the other three catalyst.

## Claims

1. A process for producing a compound of formula (I) comprising alkylation of the compound of formula (II) in the presence of a catalyst to obtain the compound of formula (I), 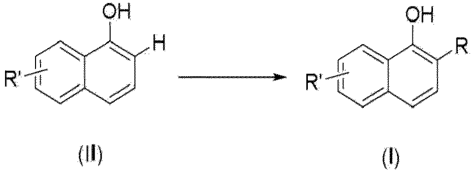
wherein R is alkyl, preferably C₁₋₆ alkyl, more preferably methyl; R' is H or 1-6 alkyls, more preferably C₁₋₆ alkyl, connected to any position on the naphthalene ring, and
wherein the catalyst contains a mixed oxide of zinc and iron.

2. The process of claim 1, wherein the alkylation is achieved by contacting a mixture that comprises the compound of formula (II) and an alcohol in gas phase with the catalyst.

3. The process of claim 2, wherein the alcohol is an aliphatic, primary or secondary alcohol, either linear or branched, and with 1 to 6 carbon atoms in the hydrocarbon chain, or mixture thereof, preferably is selected from the group consisting of methanol, ethanol, 2-propanol, 1-propanol, 1-butanol and 2-butanol.

4. The process of any one of claims 1 to 3, wherein the oxide of iron is iron oxides, preferably Fe₂O₃, Fe₃O₄ and/or FeO.

5. The process of any one of claims 1 to 3, wherein the catalyst has an atomic ratio between zinc and iron (Zn/Fe) in a range from 0.1 to10, preferably from 0.25 to 5, and more preferably from 0.5 to 4.

6. The process of any one of claims 1 to 3, wherein the catalyst is represented by the formula of ZnₓFe_{y}O_{z} wherein x is an integer from 1 to 3, preferably is 1; y is an integer from 1 to 4, preferably is 2; and z is an integer from 1 to 10, preferably is 4.

7. The process of any one of claims 1 to 3, wherein the catalyst is prepared from raw materials containing iron and raw materials containing zinc.

8. The process of claim 7, wherein the raw materials containing iron is selected from salts and oxides of iron, preferably ferric oxide, ferrous oxide, maghemite, magnetite, hematite, iron chlorides, iron nitrates and mixtures thereof.

9. The process of claim 7, wherein the raw materials containing zinc is selected from salts and oxides of zinc, preferably zinc oxide, zinc chloride and zinc nitrate.

10. The process of claim 7, wherein the catalyst is subjected to a thermal treatment at a temperature from 450 °C to 700 °C.

11. The process of any one of claims 1 to 3, wherein the total contact time, understood as the ratio between the volume of catalyst and the total volumetric flow-rate of the mixture and carrier gas, is from 0.1 seconds to 10 seconds, preferably from 0.5 to 5 seconds.

12. The process of any one of claims 1 to 3, wherein the molar ratio between the mixture in the gas phase and the total feed to the reactor is from 5% to 80%, preferably from 10% to 60%, and more preferably from 15% to 50%.

13. The process of any one of claims 1 to 3, wherein the reaction is carried out at from 300 °C to 500 °C, preferably from 350 °C to 450 °C, and more preferably from 360 °C to 390 °C.
